# EUROPEAN PATENT APPLICATION

(11) **EP 4 212 108 A1**
(43) Date of publication of application: **19.07.2023**
(21) Application number: 23152094.1
(22) Date of filing: 17.01.2023
(51) Int. Cl.: A61B 17/14, A61B 17/15, A61B 17/17

(54) **IMPROVEMENTS IN AND RELATING TO SURGICAL SAWS**

(30) Priority: 17.01.2022 GB 202200513
(71) Applicant: DePuy Ireland Unlimited Company, County Cork (IE)
(72) Inventor: BARNETT, James, Leeds, LS11 8DT (GB); BOOTH, Kevin, Leeds, LS11 8DT (GB); BOWYER, Emma, Leeds, LS11 8DT (GB); FREEMAN, Robert, Leeds, LS11 8DT (GB); RADCLIFFE, Sarah, Leeds, LS11 8DT (GB); REASON, Mark, Leeds, LS11 8DT (GB)
(74) Representative: Murgitroyd & Company

(57) **Abstract**

A surgical cutting tool and a kit incorporating the cutting tool are provided with the cutting tool comprising: a proximal end portion, the proximal end portion providing a connection location; a distal end portion, the distal end portion providing a cutting head; wherein the cutting head includes a body element, the body element includes a toothed section and the toothed section is provided with a plurality of teeth elements, at least two rows of teeth elements being provided, a plurality of the teeth elements each including an inclined face on a distal side or proximal side of the teeth element and a further inclined face on the other side of the teeth element, the inclined face of a teeth element facing across the cutting head and also face along the cutting head. The tool provides advances in easy of use in tricky sites and in controlling the dept of cutting conducted.

## Description

### TECHNICAL FIELD

This disclosure concerns improvements in and relating to cutting tools, such as surgical saws, and surgical methods using such cutting tools, particularly, but not exclusively, in relation to trial or implant site preparation.

### BACKGROUND

It is known to use reciprocating saws to cut tissue and bone in surgical applications. Some uses are intended to form a particular shape and/or configuration of cut for subsequent use. One such application is the preparation of a site, where the shape and/or configuration of the cut receives an inserted component to assist with the positioning and retention of that component.

Careful and controlled provision of the cut is necessary for it to have the desired shape and/or configuration. Existing designs may provide compromised performance, for instance with respect to accuracy or efficiency when providing the cut.

The present disclosure may provide improvements in the accuracy of the shape and/or configuration provided compared with that sought. The present disclosure may provide improvements in the efficiency of preparation of the shape and/or configuration. The present disclosure may decrease the time necessary to achieve the shape and/or configuration. The present disclosure may improve the efficiency with which bone is removed during cutting and/or the effectiveness of lavages applied to the cut site.

### SUMMARY

According to a first aspect of the disclosure there is provided a surgical cutting tool. The cutting tool may comprise a proximal end portion. The proximal end portion may provide a connection location. The cutting tool may comprise a distal end portion. The distal end portion may provide a cutting head. The cutting head may include a body element. The body element may include a toothed section. The toothed section may be provided with a plurality of teeth elements. Two rows of teeth elements may be provided.

The cutting tool may be a saw. The cutting tool may be a reciprocating cutting tool, such as a reciprocating saw.

The proximal end portion may be provided in the most proximal quarter of the length of the cutting tool, potentially considered along a longitudinal axis of the cutting tool. The axis may be a anterior-posterior axis in use.

The longitudinal axis of the cutting tool may be parallel to the axis of reciprocation of the cutting tool, in use. The longitudinal axis of the cutting tool may extend through the middle of the proximal end portion, potentially considered in terms of depth and/or thickness, and at least parallel to the axis of reciprocation of the cutting tool, in use.

Depth may be considered as the patient proximal-distal axis in use and/or thickness may be considered as the patient lateral-medial axis in use.

An intermediate portion may be provided between the proximal end portion and the distal end portion. The intermediate portion may provide at least a half of the length of the tool, potentially considered along a longitudinal axis of the cutting tool, from a proximal end of the cutting tool to a distal end of the cutting tool.

The intermediate portion may include a stem. The intermediate portion may be elongate, for instance an elongate stem. The intermediate portion may include a proximal section having an equivalent depth and/or thickness to at least a part of the proximal end portion. The intermediate portion may include an intermediate section and/or distal section having a reduced depth and/or thickness to a least a part of the proximal end portion. The intermediate portion may taper towards the distal end of the intermediate portion, for instance the depth may taper, potentially with the thickness consistent.

The proximal end of the intermediate portion may be adjacent, for instance contiguous with, the distal end of the proximal end portion.

The distal end of the intermediate portion may lead to, may be adjacent to, or could be contiguous with, the distal end portion. The distal end of the intermediate portion may be adjacent, for instance contiguous with, the cutting head, for instance a connection portion of the cutting head.

The connection portion of the cutting head may be provided intermediate the intermediate portion and the cutting head. The connection portion may be a neck portion. The connection portion may be an offset portion, for instance with the longitudinal axis of the connection portion at an angular offset compared with the longitudinal axis of the cutting tool and/or intermediate portion and/or proximal end portion and/or the cutting head. The longitudinal axis of the connection portion may be the axis extending along its middle when considered in terms of depth and thickness. The angular offset may be between 20° and 70°, for instance between 35° and 55°. The connection portion may have a reduced depth and/or thickness compared to a least a part of the intermediate portion.

The intermediate portion and the proximal end portion may extend in a common longitudinal direction, for instance along a common longitudinal axis, such as the longitudinal axis of the cutting tool.

The distal end portion may be provided in the most distal quarter of the length of the cutting tool, potentially considered along a longitudinal axis of the cutting tool.

The cutting head may include a body element. The cutting head may include a toothed section, for instance provided with a plurality of teeth elements. The cutting head may include a projecting element. The projecting element could be provided by the connection portion or at a junction between the connection portion and the body element.

The body element may be adjacent, for instance contiguous with the connection portion. The body element may provide the toothed section, for instance provided with a plurality of teeth elements. The toothed section may be provided on a lower surface of the body element, for instance an inferior surface in use.

The body element may be provided with a distal surface, for instance extending from a part of the connection portion to a part of the toothed section, such as the distal part of the connection portion and the distal part of the toothed section. The body element may be provided with an upper surface, for instance a superior surface in use. The upper surface may be provided intermediate the connection portion and the distal surface of the body element. The body element may be provided with a proximal surface, for instance extending from a part of the connection portion to a part of the toothed section, such as the proximal part of the connection portion and the proximal part of the toothed section.

The body element may be provided with one or more side surfaces. One of the side surfaces may be a lateral surface in use and another of the side surfaces may be a medial surface in use. One or both side surfaces may extend between the distal surface of the body element and the proximal surface of the body element.

The body element may have a thickness less than or equal to the thickness of the connection portion and/or intermediate portion and/or proximal end portion. The body element may have a consistent thickness. The side surfaces of the body element may be planar and/or may be parallel to one another.

The distal surface of the body element and/or the proximal surface of the body element may be planar. The distal surface of the body element and the proximal surface of the body element may be non-parallel surfaces.

The body element may be flared. The body element may flare outwardly along the longitudinal axis of the cutting tool, between an upper portion of the body element and a lower portion of the body element, for instance between a superior portion and an inferior portion, in use. The body element may be flared in terms of its extent relative to the longitudinal axis of the cutting tool. The body element may not be flared perpendicular to the longitudinal axis of the cutting tool or in terms of its thickness, for instance anterior-posterior extent.

The body element may have a first cross-section in a plane, for instance Y-Y, parallel to the longitudinal axis, for instance X-X, of the cutting tool, proximal to the connection portion and a second cross-section, for instance in plane Z-Z, parallel to both the first plane and the longitudinal axis plane, such as X-X and Y-Y, distal to the connection portion. The first cross-section and the second cross-section may have the same lateral extent, for instance direction S-S, such as an anterior-posterior extent. The second cross-section may have a greater longitudinal extent, for instance direction L-L, than the first cross-section. The lateral extent of the first cross-section and the second cross-section may correspond to the width of the intermediate portion and/or the width of the connection portion.

A plurality of the teeth elements may be provided in a common plane. Greater than 50% of the teeth element may be provided in a common plane. Greater than 80% of the teeth may be provided in a common plane. All of the teeth elements may be provided in a common plane.

The common plane may be substantially parallel or parallel to the axis or plane in which the cutting tool reciprocates in use. The common plane may be lower, for instance inferior is use, that the longitudinal axis of the cutting tool. The common plane may be lower, for instance inferior in use, compared with the body element and/or connection portion and/or intermediate portion and/or proximal end portion.

One or more or all of the teeth elements may have a tip. One or more or all of the teeth elements may have a base profile, for instance where the teeth element meets the body element.

The tips of the plurality of teeth elements in the common plane may be provided in the common plane. The base profiles of the plurality of teeth elements provided in the common plane may be provided in the common plane. One part of the teeth elements, such as the tip, may be provided in a first common plane. A second part of the teeth elements, such as the base profile, may be provided in a second common plane.

In use, a plurality of the teeth elements, potentially more that 50% of the teeth elements, optionally more than 80% of the teeth elements and potentially all of the teeth elements may have the same inferior extent, for instance inferior extent into the bone of the patient.

The toothed section may include a plurality of rows of teeth elements. Two rows of teeth elements may be provided.

A first row of teeth elements may be formed by teeth elements having the same orientation as each other. Another row of teeth elements may be formed by teeth elements having the same orientation as each other, but having a different orientation to the first row of teeth elements. The another row of teeth elements may have one or more or all teeth elements having an orientation which is the mirror image of the orientation of one or more or all of the teeth element of the first row.

One or more of the teeth elements of one row may be at least partially interdigitated with one or more of the teeth elements of another row. At least two teeth elements, for instance at least three teeth elements, of one row may be at least partially interdigitated with two or more, for instance three or more, teeth elements of another row. All of the teeth elements of one row may be at least partially interdigitated with all of the teeth elements of another row.

One or more of the teeth elements of one row may at least partially overlap with one or more of the teeth elements of another row. At least two teeth elements, for instance at least three teeth elements, of one row may at least partially overlap with two or more, for instance three or more, teeth elements of another row. All of the teeth elements of one row may at least partially overlap with all of the teeth elements of another row. The overlap may be with respect to a distal-proximal axis or view, such as an anterior-posterior axis or view, in use.

One or more of the teeth elements of one row may be staggered relative to one or more of the teeth elements of another row. At least two teeth elements, for instance at least three teeth elements, of one row may be staggered relative to two or more, for instance three or more, teeth elements of another row. All of the teeth elements of one row may be staggered relative to all of the teeth elements of another row. The stagger may be with respect to a distal-proximal axis or view, such as an anterior-posterior axis or view, in use.

From the distal side of the cutting head towards the proximal side of the cutting head and/or vice-versa, teeth elements may be encountered in alternating positions, for instance on alternating sides of the cutting head.

The teeth elements may have a three-dimensional profile. The teeth element may include one or more cutting edges and/or cutting surfaces as part of the profile. The teeth elements may have a pyramidal profile. The pyramidal profile may extend from a triangular base profile.

One or more or all of the teeth elements may include a single edge extending from a base profile to a tip. The edge extending from the base profile to a tip across the width of the cutting head may consist of a single edge. One or more or all of the teeth elements may include multiple single edges, for instance three edges, extending from a base profile, for instance a triangular base profile, to a tip. The one or more single edges may be straight edges. The one or more single edges may be edges free from intersections of different edges or sections of edge with one another. The one or more single edges may be edges free from intersections of different angled parts of the edge.

One or more or all of the teeth elements may be defined by three intersecting triangular faces. One or more or all of the teeth element may be defined by the intersection of a triangular side wall face and two triangular inclined faces.

One or more or all of the teeth elements may include an inclined face. One or more or all of the teeth elements may further include a further inclined face. The inclined face may be provided on the distal side of a teeth element or on the proximal side of the teeth element. Where provided, the further inclined face may be provided on the other side of a teethed element relative to the inclined face.

An inclined face of a teeth element may face across the cutting head and also face along the cutting head. An inclined face of a teeth element and a further inclined face of that teeth element may face across the cutting head and also face along the cutting head. A plurality or all of the teeth elements may be provided with such inclined faces and/or further inclined faces.

The inclined face and the further inclined face of a teeth element may both face across the cutting head, for instance at the same angle, but face in opposite directions along the cutting head.

An inclined face of a teeth element may be inclined relative to the inferior-superior direction and also inclined relative to the lateral-medial direction. An inclined face may be inclined relative to the inferior-superior direction and also inclined relative to the anterior-posterior direction. An inclined face may be inclined relative to the inferior-superior direction and also inclined relative to the anterior-posterior direction and also inclined relative to the lateral-medial direction. An inclined face of a teeth element and a further inclined face of the teeth element may be inclined relative to the inferior-superior direction and also inclined relative to the lateral-medial direction. An inclined face and a further inclined face of the teeth element may be inclined relative to the inferior-superior direction and also inclined relative to the anterior-posterior direction. An inclined face and a further inclined face of the teeth element may be inclined relative to the inferior-superior direction and also inclined relative to the anterior-posterior direction and also inclined relative to the lateral-medial direction.

The inclined face and the further inclined face of a teeth element may both have the same inclination relative to the inferior-superior direction and/or same inclination relative to the anterior-posterior direction and/or inclination relative to the lateral-medial direction, for instance at the same angle, but face in opposite directions along the cutting head and/or across the cutting head.

For the non-distal end and/or non-proximal end teeth elements in particular, an Isosceles triangle base profile may be provided. For the distal end and/or proximal end teeth elements in particular, a right-angled triangle base profile may be provided. A side wall of the teeth elements may have an Isosceles triangle face profile. One or two or more inclined faces of the teeth element may have a right-angle triangle profile. An end wall of the teeth elements may have a right-angled triangle profile.

One or more transition surfaces may be provided between a teeth element and an adjoining element, for one or more or all of the teeth elements. The one or more transition surfaces may extend from an edge of one teeth element to the edge of an adjoining teeth element. The one or more transition surfaces may be planar. The one or more transition surfaces may be curved. The one or more transition surfaces may have a distal-proximal extent which is less than 25% of the maximum distal-proximal extent of the teeth element.

An inclined face of one teeth element and a further inclined face of an adjacent teeth element may define a channel between the two teeth elements. The channel may be partially defined by a transition surface between the two teeth elements. The channel may be angled relative to the longitudinal axis of the cutting tool and/or of the distal end portion and/or the cutting head and/or the axis of reciprocation, in use. The angle may be between 15° and 45°, for instance between 25° and 35°.

The channel between one pair of teeth elements may be angled in the opposite direction to the channel between the next pair of teeth elements, for instance formed by one of those teeth elements of the one pair and the next teeth element to it. The channels may alternate in the direction that they are angled progressing from the proximal side to the distal side of the cutting head.

The cutting head and particularly the plurality of teeth elements, may define a gap between the teeth elements in a cross-sectional profile across a plane. The plane may be perpendicular to the longitudinal axis of the cutting tool and/or perpendicular to the common plane of the teeth elements and/or perpendicular to the axis of reciprocation of the cutting tool, in use.

The gap may extend between the teeth elements of one row of teeth elements and the teeth elements of another row of teeth elements.

The gap may extend from the distal side of the cutting head to the proximal side of the cutting head, when viewed along the longitudinal axis.

The gap may have a continuous presence at all positions for the plane along the axis. The gap, viewed from a position for the plane at the distal end or the proximal end of the cutting head, may define a gap minimum apparent cross-section. The gap minimum apparent cross-section may be a projection of the gap from the distal end to the proximal end or vice-versa. The gap cross-section may exceed the gap minimum apparent cross-section at one or more locations along the axis.

The gap may be a gullet.

The gap may be a single gap only.

The gap may be defined by the projection of a portion of the edge of the teeth elements in one row of teeth elements and the projection of a portion of the edge of the teeth elements in another row of teeth elements. The gap may be further defined by the intersection of the projection of the portion of the edge of the teeth elements in one row of teeth elements and the projection of the intersection of the portion of the edge of the teeth elements in another row of teeth elements. The edge may be linear when viewed in the plane. A single linear edge of one teeth element and a single linear edge of another teeth element may define the shape of the gap, particularly the minimum cross-section thereof. The gap may be a V-shape, particularly an inverted V-shape, when viewed from the plane.

One or more or all of the teeth elements may have three or more teeth faces provided between the base profile and the tip.

One or more of all of the teeth elements may include a side face. The side face may extend to a side surface of the body element, for instance in a contiguous manner. One or more or all of the side faces may be substantially parallel or parallel to their respective side face. One or more of all of the side faces may be coplanar with a side face of the body element.

A first teeth element type may be provided which has a different profile compared with a second teeth element type.

The first teeth element type may be provided as the most distal and/or most proximal teeth element in the plurality of teeth elements, considered relative to the distal and/or proximal ends of the cutting tool.

The second teeth element type may provide one or more or all of the plurality of teeth elements provided intermediate the most distal and/or most proximal teeth element in the plurality of teeth elements.

The first teeth element type may include teeth element[s] which are mirror images of other teeth element[s] in the first teeth element type, for instance the most distal teeth element may be a mirror image of the most proximal teeth element.

The first teeth element type may include a side face. The side face may extend to a side surface of the body element, for instance in a contiguous manner. The side face and the side surface may be coplanar.

The first teeth element type may include an end face. The end face may extend to a distal surface of the body element, for instance in a contiguous manner. The end face and the distal surface may be coplanar. The end face may extend to a proximal surface of the body element, for instance in a contiguous manner. The end face and the proximal surface may be coplanar.

The first teeth element type may include one or more inclined faces. The first teeth element type may consist of only a single inclined face, a side face and an end face. The one or more inclined faces may be planar. The one or more inclined faces may be inclined relative to the side face and/or end face of the first teeth element type.

The first teeth element type may include one or more edges. The first teeth element type may include one or more edge types, such as a first edge, a second edge, a third edge and a fourth edge.

The first teeth element type may include a first edge. The first edge may be defined between a side face and an end face. The first edge may extend between a tip and the side surface of the body element. The first edge may be aligned with the plane of the side face and/or the plane of the end face. The first edge may be linear. The first edge may extend in a superior-inferior direction, in use. The first edge may be provided at a single angle.

The first teeth element type may include a second edge. The second edge may be defined between an end face and an inclined face. The second edge may extend between a tip and the distal surface of the body element or the proximal surface of the body element. The second edge may be inclined relative to the plane of the side face and/or the plane of the end face. The second edge may be linear. The second edge may extend in a superior-inferior direction, in use, but be non-perpendicular to the superior-inferior direction, in use. The second edge may be provided at a single second angle. The second edge may extend across the width of the body element or a substantial part thereof.

The first teeth element type may include a third edge. The third edge may be defined between a side face and an inclined face. The third edge may extend between a tip and the lower or inferior surface of the body element. The third edge may be aligned relative to the plane of the side face and/or inclined relative to the plane of the end face. The third edge may be linear. The third edge may extend in a superior-inferior direction, in use, but be non-perpendicular to the superior-inferior direction, in use. The third edge may be provided at a single third angle. The third edge may extend across the width of the body element or a substantial part thereof.

The first teeth element type may include a fourth edge. The fourth edge may be defined between an inclined face and the body element. The fourth edge may extend between a junction between the second edge and the lower or inferior surface of the body element and a junction between the third edge and the lower or inferior surface of the body element. The fourth edge may be angled relative to the plane of the side face and/or angled relative to the plane of the end face. The fourth edge may be linear. The fourth edge may extend in a lateral-medial and/or anterior-posterior direction, in use, but be substantially perpendicular to the superior-inferior direction, in use. The fourth edge may be provided at a single fourth angle. The fourth edge may extend across the width of the body element or a substantial part thereof.

The second teeth type may include teeth element[s] which are mirror images of other teeth element[s] in the second teeth element type. For instance, one or more or all of the teeth elements in one row of teeth elements may be mirror image of one or more or all of the teeth elements in another row of teeth elements. All of the teeth elements in a first row of teeth elements may be mirror images of all of the teeth elements in another row of teeth elements, bar the most distal teeth element and/or the most proximal teeth element.

The second teeth element type may include a side face. The side face may extend to a side surface of the body element, for instance in a contiguous manner. The side face and the side surface may be coplanar.

The second teeth element type may include two or more inclined faces. The second teeth element type may consist of only two inclined faces and a side face. The two or more inclined faces may be planar. The two or more inclined faces may be inclined relative to the side face and/or the other of the two or more inclined faces of the second teeth element type.

The second teeth element type may include one or more edges. The second teeth element type may include one or more edge types, such as a second edge, a third edge, a further third edge, a fourth edge and a further fourth edge. The second teeth element type may lack one or more edge types, such as a first edge.

The second teeth element type may include a second edge. The second edge may be defined between a first inclined face and a further inclined face. The second edge may extend between a tip and the lower or inferior surface of the body element. The second edge may be inclined relative to the plane of the side face and/or the plane of the end face. The second edge may be linear. The second edge may extend in a superior-inferior direction, in use, but be non-perpendicular to the superior-inferior direction, in use. The second edge may be provided at a single second angle. The second edge may extend across the width of the body element or a substantial part thereof.

The second teeth element type may include a third edge and/or a further third edge. The third edge and/or the further third edge may be defined between a side face and an inclined face. The third edge and/or the further third edge may extend between a tip and the lower or inferior surface of the body element. The third edge and/or the further third edge may be aligned relative to the plane of the side face and/or inclined relative to the plane of the end face. The third edge and/or the further third edge may be linear. The third edge and/or the further third edge may extend in a superior-inferior direction, in use, but be non-perpendicular to the superior-inferior direction, in use. The third edge and/or the further third edge may be provided at a single third angle. The third edge and/or the further third edge may extend across the width of the body element or a substantial part thereof.

The second teeth element type may include a fourth edge and/or a further fourth edge. The fourth edge and/or the further fourth edge may be defined between an inclined face and the body element. The fourth edge and/or the further fourth edge may extend between a junction between the second edge and the lower or inferior surface of the body element and a junction between the third edge and the lower or inferior surface of the body element. The fourth edge and/or the further fourth edge may be angled relative to the plane of the side face and/or angled relative to the plane of the end face. The fourth edge and/or the further fourth edge may be linear. The fourth edge and/or the further fourth edge may extend in a lateral-medial and/or anterior-posterior direction, in use, but be substantially perpendicular to the superior-inferior direction, in use. The fourth edge and/or the further fourth edge may be provided at a single fourth angle. The fourth edge and/or the further fourth edge may extend across the width of the body element or a substantial part thereof.

The body element may be provided with a projecting element, for instance a lateral projection. The projecting element could be provided by the connection portion or at a junction between the connection portion and the body element.

The projecting element may extend from one side of the cutting tool, for instance the body element and particularly a side surface of the body element. A lateral projection from both sides of the cutting tool could be provided.

The projecting element may extend in a medial direction, is use, but could extend in a lateral direction, in use.

The projecting element projection may be provided with a lower surface, for instance an inferior surface in use, which includes a planar portion. A substantial part, for instance greater than 70%, of the lower surface may be planar. The planar portion may be substantially parallel or parallel to the longitudinal axis of the cutting tool and/or to the common plane of the teeth elements and/or to the axis of reciprocation of the cutting tool in use. The planar portion may be substantially perpendicular or perpendicular to the adjacent side surface of the body element. Substantially parallel or substantially perpendicular may be +/- 5°.

The projecting element may be provided with a proximal end surface and a distal end surface, for instance curved surfaces extending between the lower surface and an upper surface. The upper surface may be at least partially planar, for instance parallel to the planar portion of the lower surface.

The distance between the planar portion of the lower surface and one or more of the tips may control the cutting depth of the cutting tool. In particular, the distance between the planar portion of the lower surface and the furthest common plane in which tips of the teeth elements lie may define the cutting depth of the cutting tool. The cutting depth of the tool may be defined by the perpendicular separation of the planar portion of the lower surface and the common plane of the tips of the teeth elements.

The connection location may, in use, connect the cutting tool to an operative unit. At least a part of the connection location and/or proximal end portion may be received in an operative unit, when the cutting tool is operatively connected to the operative unit.

The operative unit may be a power tool. The operative unit may provide reciprocating drive to the cutting tool.

The connection location may include one or more locations which provide one or more reduced depth parts and/or one or more reduced thickness parts when compared with one or more other locations. The connection location may include one or more locations which provide one or more increased depth parts and/or one or more increased thickness parts when compared with one or more other locations.

The connection location may be provided in the most proximal quarter of the length of the cutting tool, potentially considered along a longitudinal axis of the cutting tool.

The first aspect of the disclosure may include any of the features, options or possibilities set out elsewhere in this document, including in the other aspects of the disclosure.

According to a second aspect of the disclosure there is provided a kit comprising:
a cutting tool, the cutting tool comprising:
   a proximal end portion, the proximal end portion providing a connection location;
   a distal end portion, the distal end portion providing a cutting head;
   wherein the cutting head includes a body element, the body element includes a toothed section and the toothed section is provided with a plurality of teeth elements, two rows of teeth elements being provided; and
one or more further surgical components.

The one or more surgical components may include a cutting template.

The cutting template may comprise an at least partially planar under surface or inferior surface. The under surface or inferior surface may be configured to contact a surgical site, such as a tibial surface or tibial plateau.

The cutting template may comprise an at least partially planar upper surface or superior surface. The upper surface or superior surface may be configured to contact the cutting tool, particularly a projecting element, such as a lateral projection, thereon.

The cutting template may be provided with a through slot. The through slot may have an anterior-posterior extent, in use. The anterior-posterior extent may be greater than the maximum anterior-posterior extent of the cutting head. The greater anterior-posterior extent of the through slot may accommodate the reciprocating movement of the cutting head. The through slot may have a lateral-medial extent, in use. The lateral-medial extent of the through slot may exceed the lateral-medial extent of the cutting head. The lateral-medial extent of the cutting head may be at least 95% of the lateral-medial extent of the through slot. The lateral-medial extent of the of the cutting head may correspond to the lateral-medial extent of the through slot, less a clearance.

The one or more surgical components may include an operative unit, such as a power tool, for instance for connection to the cutting tool.

The second aspect of the disclosure may include any of the features, options or possibilities set out elsewhere in this document, including in the other aspects of the disclosure.

According to a third aspect of the disclosure there is provided a method of preparing a surgical site, the method comprising:
a) providing access to the surgical site;
b) introducing a cutting tool to the surgical site;
c) cutting material at the surgical site;

wherein the cutting tool comprises:
   a proximal end portion, the proximal end portion providing a connection location;
   a distal end portion, the distal end portion providing a cutting head;
wherein the cutting head includes a body element, the body element includes a toothed section and the toothed section is provided with a plurality of teeth elements, two rows of teeth elements being provided.

The method may further comprise connecting the cutting tool to an operative unit, such as a power tool.

The method may further comprise selecting one or more surgical components for use in the method, potentially from amongst a set of such further components. The method may include selecting a cutting template.

The method may include inserting the cutting template into the surgical site. The method may include placing the cutting template on a surface in the surgical site, such as a tibial surface. The method may include contacting an at least partially planar under surface or inferior surface with the surface in the surgical site. The cutting template may be introduced and/or placed prior to step b).

The method may include placing the cutting head on a part of the surgical site accessible through a through slot in the cutting template. The method may include moving, for instance reciprocating, the cutting head relative to the surgical site. The method may include moving, for instance reciprocating, the cutting head relative to the surgical site within the through slot of the cutting template to provide cutting of the surgical site. The method may include moving the cutting head in an anterior-posterior alignment. The method may include the cutting template limiting movement of the cutting head in an anterior-posterior alignment. The method may include the cutting template substantially eliminating movement of the cutting head in a lateral-medial alignment.

The method may include preparing the surgical site to receive a further surgical component, such as a trial component and/or an implanted component. The trial component and/or implanted component may have a projecting element, such as a keel, on a lower surface and/or inferior surface. The method may include forming a slot in the surgical site to receive the projecting element. The cutting template may have a through slot dimensioned to provide the desired slot in the surgical site, particularly in the anterior-posterior and/or lateral-medial directions.

The method may include continuing with the cutting of the surgical site until a projection element on the cutting head contacts the upper surface or superior surface of the cutting template. The method may include continuing with the cutting of the surgical site until the projection element has a maximum extent of contact with the upper surface or superior surface of the cutting template. The method may include continuing with the cutting of the surgical site until the contact between the projection element and the cutting template prevents the depth of the slot in the surgical site increasing. The method may continue with the cutting of the surgical site until the maximum depth and/or maximum lateral-medial extent and/or maximum anterior-posterior extent for the slot in the surgical site is provided.

The method may include removal of the cutting template once the slot in the surgical site is formed.

The method may include inserting a trial component into the surgical site, potentially with a projection of the trial component inserted into the slot in the surgical site. The method may include removal of the trial component from the surgical site.

The method may include inserting an implant into the surgical site, potentially with a projection of the implant inserted into the slot in the surgical site.

The method include one or more surgical method steps, particularly provided before step c), selected from: pre-surgical planning; resection, such as tibial resection; jog alignment, such as tibial jig alignment; provision of a planar surface at the surgical site, for instance tibial resection to prepare a tibial plateau; assessment of the surgical site, for instance for slope; assessment of the size of the surgical site or part thereof, for instance a tibial trial can be used to confirm the component size; balancing; resection, such as femoral resection; sizing and rotation, such as femoral sizing and rotation; reduction, such as trial reduction.

The method may include one or more further surgical method steps, particularly provided during step c) or afterwards, selected from: application of lavage; provision of fixing locations for the trial component and/or implant; fixing of the trial component and/or implant; completion of the surgery.

The third aspect of the disclosure may include any of the features, options or possibilities set out elsewhere in this document, including in the other aspects of the disclosure.

### BRIEF DESCRIPTION OF THE DRAWINGS

Various embodiments of the disclosure will now be described, by way of example only and with reference to the accompanying drawings in which:
Figure 1 is a side view of a cutting tool according to the disclosure;
Figure 2 is plan view from beneath of the cutting tool of Figure 1;
Figure 3 is a distal end view of the cutting tool of Figure 1 and Figure 2;
Figure 4 is a side view of a head detail for a cutting tool according to the disclosure;
Figure 5 is a plan view from beneath of the head detail of Figure 4;
Figure 6a is a perspective view of a knee joint showing a tibial plateau to be prepared for a tibial trial with a keel;
Figure 6b shows a cutting tool according to the disclosure being used to form a cut to receive the keel of a tibial trial;
Figure 6c shows the cut formed and the tibial insert being inserted;
Figure 7 is a schematic cross-sectional view of a cut showing problem locations within the cut.

### DETAILED DESCRIPTION OF THE DRAWINGS

It is known to use reciprocating saws to cut tissue and bone in surgical applications. Some uses are intended to form a particular shape and/or configuration of cut for subsequent use. One such application is the preparation of a site, where the shape and/or configuration of the cut receives an inserted component to assist with the positioning and retention of that component. In the context of a trial implant or the actual implant, for instance a tibial implant, a cut may be provided which has the shape and/or configuration to receive the distally extending keel of the trial or implant. The cut, in the form of a suitably dimensioned slot, is formed in the tibial plateau to receive this keel. This provides and maintains the trial or implant in the correct position for other steps and use.

Existing designs of cutting tool offer impaired performance in one or more ways. For instance, the cutting tool may not provide the cut with sufficient accuracy and control. It is important for the cut to be in the correct position or configuration, such that the trial or implant has the correct position in the joint. It is important for the cut to have the correct profile or shape, such that the keel is fully received, but a secure receipt of the keel is achieved. Too much material removed or too little material removed can impair performance. There can be issues with controlling the depth of the cut and/or the completeness of material removal with existing designs.

The present disclosure may provide improvements in the accuracy of the shape and/or configuration provided compared with that sought. The present disclosure may provide improvements in the efficiency of preparation of the shape and/or configuration. The present disclosure may decrease the time necessary to achieve the shape and/or configuration. The present disclosure may improve the efficiency with which bone is removed during cutting and/or the effectiveness of lavages applied to the cut site.

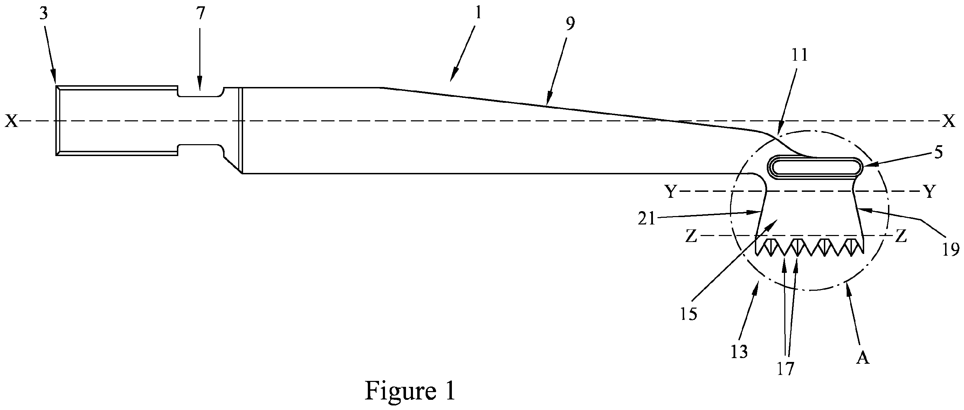

Referring to Figure 1, the cutting tool 1 is provided with a proximal end 3 and a distal end 5.

The proximal end 3 provides the connection location 7 which is used to connect the cutting tool 1 to an operative unit [not shown]. The connection location 7 can be of any suitable profile, well known in the art, to provide a stable connection to the operative unit. The operative unit may be a reciprocating drive unit for the cutting tool.

The proximal end 5 is connected to the distal end 3 by an elongate stem 9. The elongate stem 9 extends along the longitudinal axis X-X of the cutting tool 1.

The distal end 5 includes a neck portion 11 and a cutting head 13. The neck portion 11 connects the elongate stem 9 to the cutting head 13. The neck portion 11 is angled relative to the longitudinal axis X-X so as to offset the cutting head 13 relative to that axis.

The cutting head 13 has body element 15 provided with a plurality of teeth elements 17.

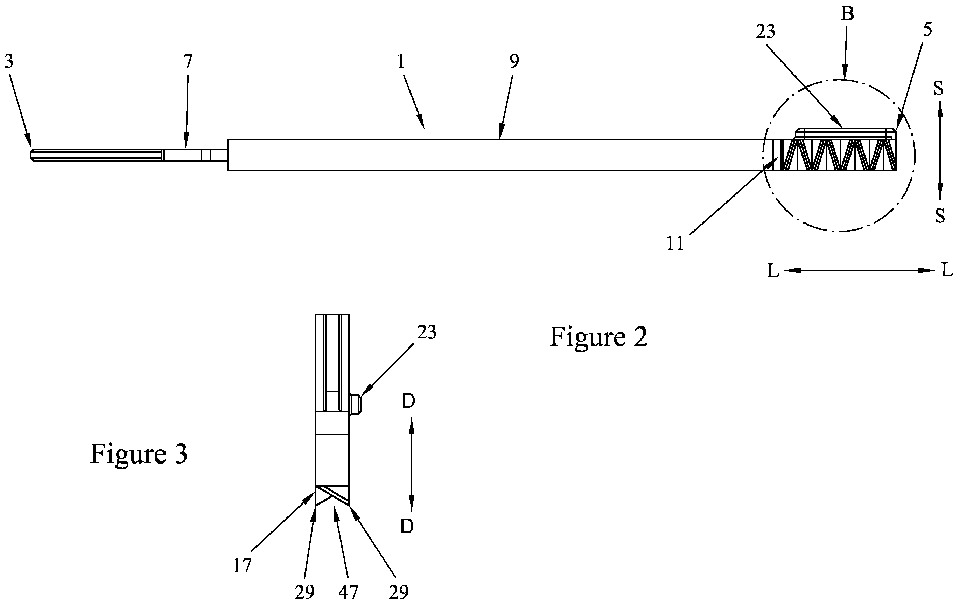

The body element 15 flares away from the neck portion 11 as the front surface 19 and the rear surface 21 are inclined relative to one another. Hence, the body element 15 has a first cross-section in plane Y-Y, parallel to the longitudinal axis X-X, proximal to the neck portion 11 and a second cross-section in plane Z-Z, parallel to both axis X-X and Y-Y, but distal to the neck portion 11. The first cross-section and the second cross-section have the same lateral extent, direction S-S in Figure 2. The second cross-section has a greater longitudinal extent, direction L-L in Figure 2, than the first cross-section. The lateral extent of the first cross-section and the first cross-section also corresponds to the width of the elongate stem 9 and the width of the neck portion 11.

The body element 15 has a lateral projection 23 best seen in Figure 2 and Figure 3. This lateral projection 23, in the form of a flange as illustrated, serves to limit the depth, D-D, that the cutting tool 1 can cut to, in a manner described in more detail below in the method section.

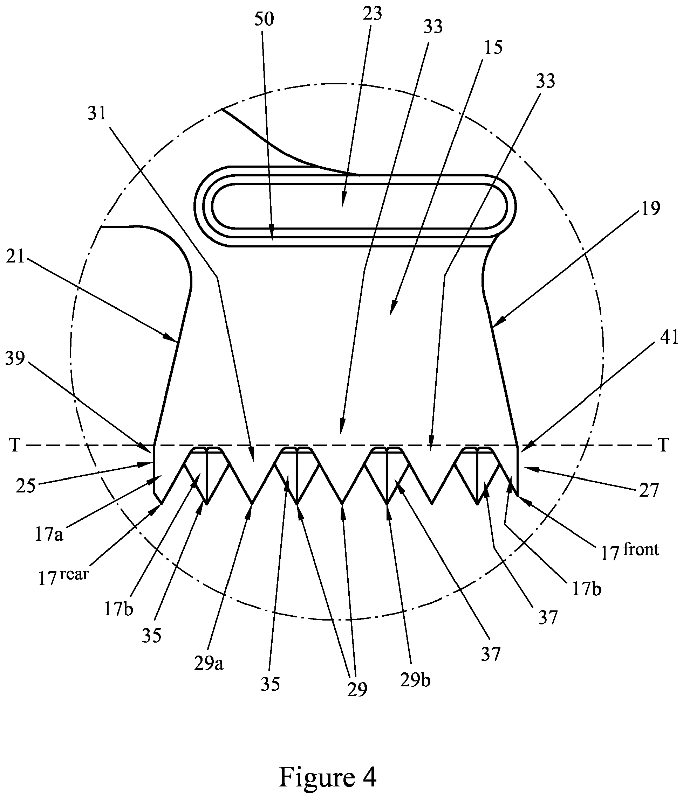

As can be seen in Figure 4, the teeth elements 17a on one side of the body element 15 are staggered with respect to the teeth elements 17b. Thus, as they progress from the proximal edge 25 to the distal edge 27, the tip 29 encountered alternates from one side of the body element 15 to the other and back again in a repeating cycle. In the illustrated form there are five tips 29a on one side and four tips 29b on the other side.

The outside face 31 of each of the teeth elements 17 is flush with the outer surface 33 of the body element 15 on both sides of the body element 15.

The teeth elements 17 can be thought of as pyramids where the outside face 31 serves to truncate or halve the pyramid. The teeth elements 17 thus have a planar triangular base profile 33 in plane T-T and an outer face 31 that is perpendicular to the planar triangular base profile 33. The other two faces of the triangular pyramid profile for the teeth elements 17 are the proximal internal triangular face 35 and the distal internal triangular face 37. The end most tooth element 17, both distally and proximally, has a different profile to the other teeth element. The proximal most tooth element 17^{rear} is further truncated by a proximal end face 39 which extends between the tip 29 of that tooth element 17^{rear} and the rear surface 21. The distal most tooth element 17^{front} is further truncated by a distal end face 41 which extends between the tip 29 of that tooth element 17^{front} and the front surface 19. This truncation further halves the tooth element 17 in each case.

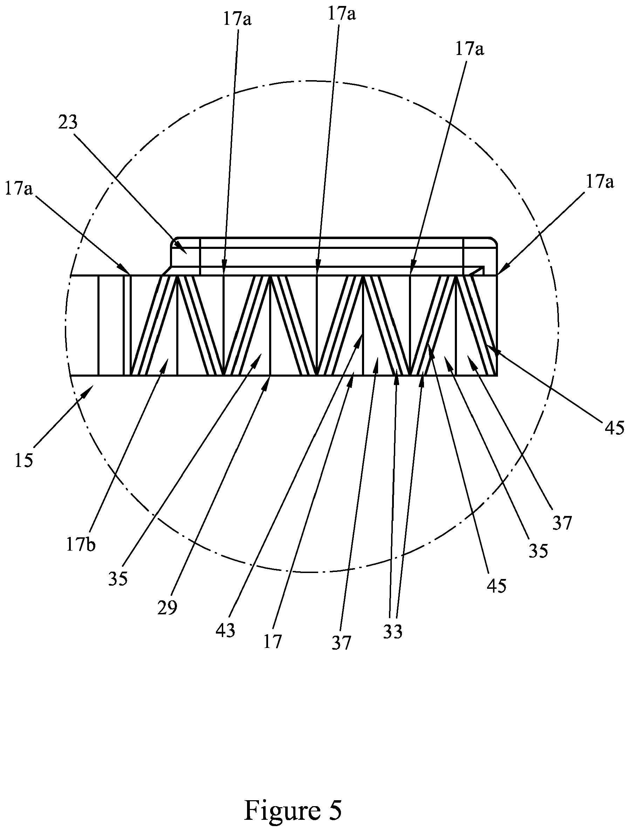

As can be seen in Figure 5, the teeth elements 17a on one side of the body element 15 are interdigitated with the teeth elements 17b on the other side of the body element 15. From its tip 29 each tooth has an edge 43 which separates the proximal internal triangular face 35 and the distal internal triangular face 37 from one another and which leads to the planar triangular base profile 33 where the tooth element 17 joins the body element 15.

Between adjoining teeth elements 17, a transition surface 45 is provided. This may be a radius between the adjoining faces of adjoining teeth elements 17; between the distal internal triangular face 37 of one tooth element 17 and the proximal internal triangular face 35 of the next tooth element 17. The distal internal triangular face 37 of one tooth element 17 could contact directly with the proximal internal triangular face 35 of the next tooth element 17 as an alternative form of transition or different transition surface 45 shapes could be used.

When viewed from the proximal end 3 [as in Figure 3] or from the distal end 5, a channel 47 extends longitudinally between the tips 29 of the teeth elements 17. This single channel 47 or gullet assists with the displacement of cut material from the cutting site.

Whilst the cutting tool is useful in various surgical procedures, one example of particular relevance is in the preparation of a tibial surface to receive a tibial trial or implant.

The method uses one of the established methodologies, for example including the necessary steps from: pre-surgical planning; tibial resection; tibial jig alignment; and tibial resection to prepare a tibial plateau. With the resected bone removed, the site can be assessed to confirm reproduction of the slope and a tibial trial can be used to confirm the component size. The method may then continue with one of the established methodologies, for example including the necessary steps from: balancing; femoral resection; femoral sizing and rotation; trial reduction; before returning to the tibia and its preparation.

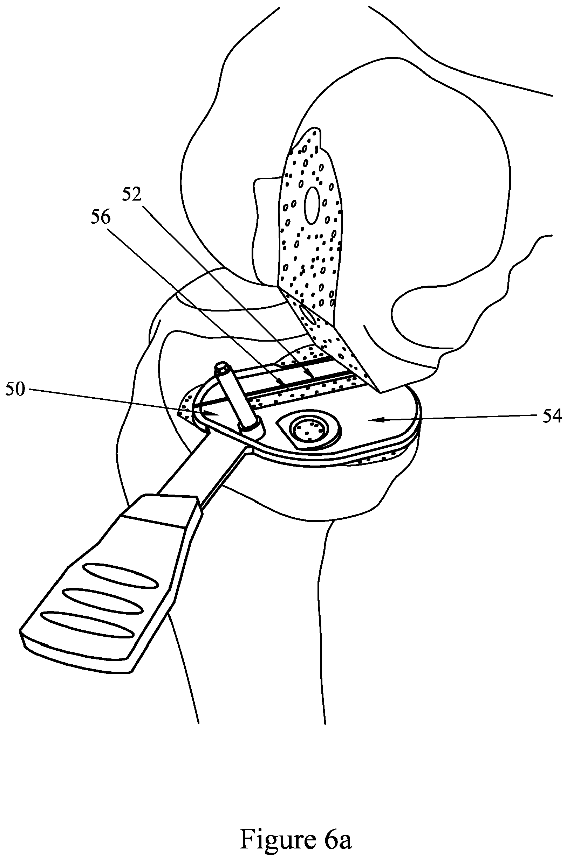

To prepare the tibia for a keel on the posterior of the tibial component to be inserted, a tibial template 50, that matches the selected tibial trial, is inserted and then tapped to ensure proper positioning and orientation on the tibial plateau 52; see Figure 6a. The tibial template 50 has a planar proximal surface 54 with a through slot 56 which exposes a part of the tibial plateau 52. The anterior-posterior dimensions of the slot 56 control the length of the cut slot 58 to be cut in the tibial plateau 52 and the lateral-medial dimensions of the slot 56 control the width of the cut slot 58 to be cut in the tibial plateau 52. The tibial template 50 by itself cannot control the depth of any cut made.

A spreader, not shown, may be used to improve access to the tibial template 50 and allow the introduction of the cutting tool 1 mounted on an operative unit. The cutting head 13 is positioned on the part of the tibial plateau 52 exposed through the tibial template 50 and the cutting tool 1 is operated in a reciprocating manner. The movement is reciprocation in the anterior-posterior directions.

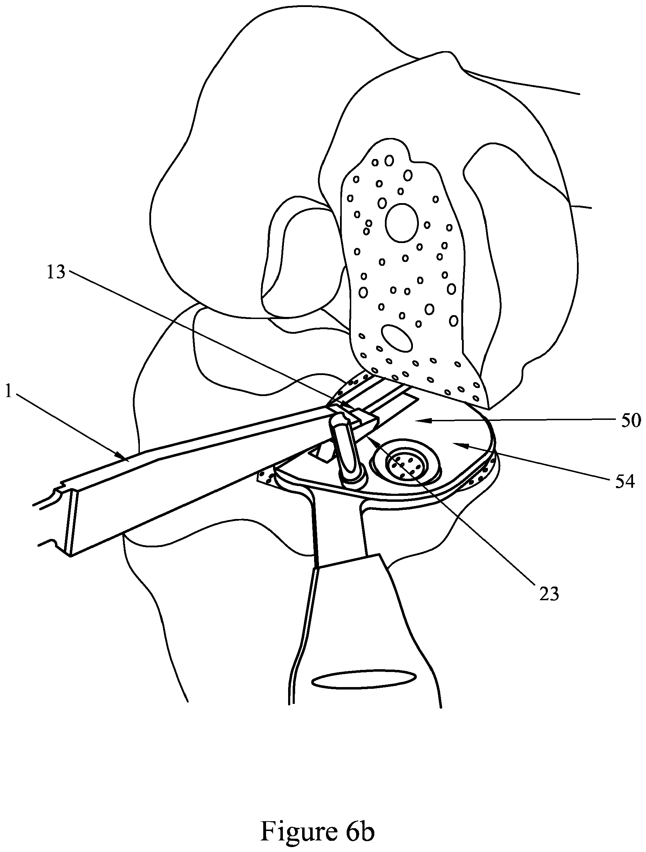

The cutting can continue, with the reciprocating motion generally parallel to the planar proximal surface 54 of the tibial template 50, as the depth of the cut slot 58 increases. As the depth increases, the lateral projection 23 on the cutting tool 1 will start to contact the tibial template 50 and will promote increasing alignment between the reciprocation and the planar proximal surface 54 of the tibial template 50; see Figure 6b.

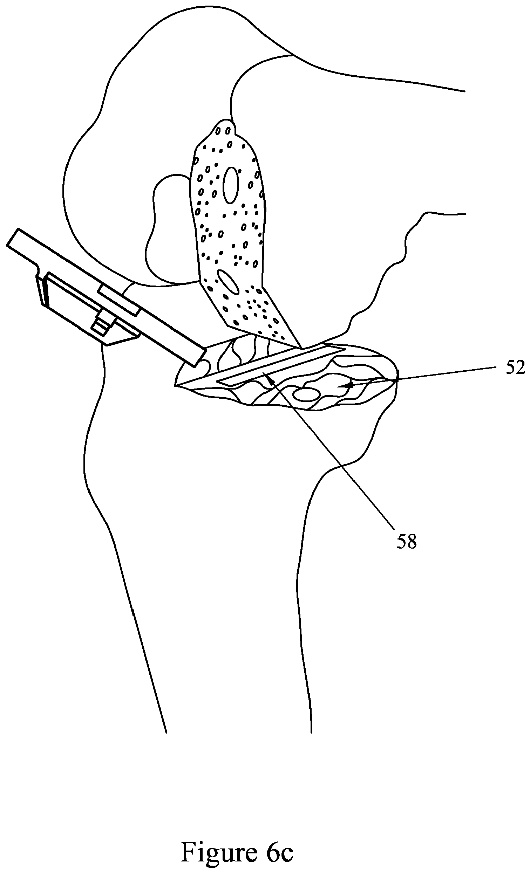

Eventually, the movement will be with the tooth element side surface 50 of the lateral projection 23 moving over the planar proximal surface 54 of the tibial template 50 and this will limit the depth of the cut slot 58 in the tibial plateau 52 and will ensure that the depth and profile more generally is consistent in the anterior-posterior and lateral-medial directions throughout the site of the cut slot 58. An optimised seat for the keel is thus provided as the cutting head 13 has a depth D-D configure to give a cut slot 58 depth as desired for the keel depth it needs to accommodate; see Figure 6c.

As cutting progresses and/or afterwards, the application of lavage may be used to remove bone fragments from the site and surrounds of the cut slot 58. With the cutting completed, the cutting tool 1 is removed and the slot is ready for the keel and further steps of the methodology.

The method may then continue with one of the established methodologies, for example including the necessary steps from: tibial trial insertion; peg hole formation; provision and fixation of the tibial implant; completion of the surgery.

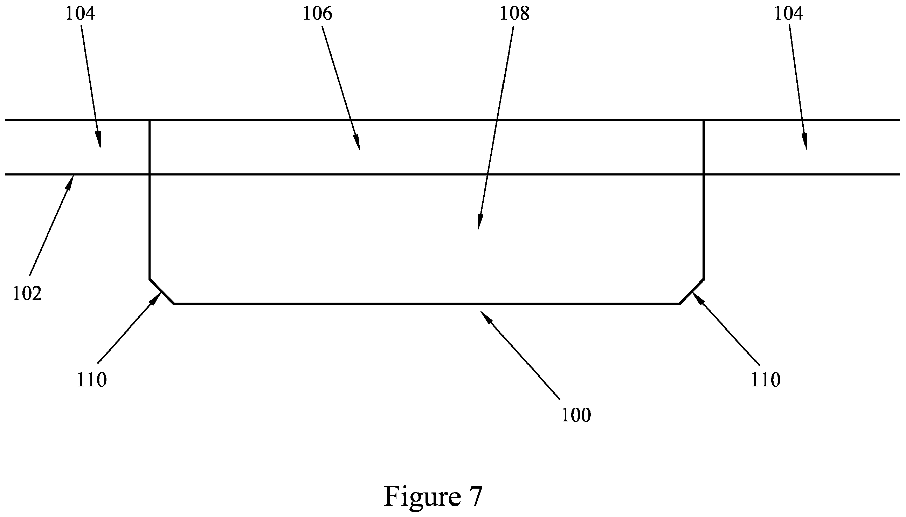

With respect to the disclosures control over and improved form for the cutting delivered to the cutting site, reference is made to Figure 7 which is a schematic illustration of a cutting site 100 in cross-sectional side view. The tibial plateau 102 has a tibial template 104 positioned on it and the through slot 106 in the tibial template 104 guides the cutting head [not shown] when in use. The slot 108 formed at the cutting site 100 can be imperfectly formed in prior art designs as the cutting head faces difficulties in accessing the limits of the cutting site. Thus, sections of material, such as those with designation 110, may be left in place even after it is believed that the slot 108 at the cutting site 100 has been completed to the desired extent. That can have a negative impact upon the receipt of the keel within the slot 108.

The cutting tool 1 of the disclosure is provided with a cutting head 13 that flares outwardly in the distal direction due to the flaring end surfaces 19 and 21. This means that the cutting head 13 is more successful in reaching the sections of material, exemplified by designation 110, and hence the slot 108 is fully completed with the desired profile to receive the keel.

The keels are generally configured with a single depth parameter used across different implants and so only a single cutting tool 1 is required for a wide variety of implants. Generally, a single width is used and that is controlled by the template 104. Different implants may have different lengths of keel and that is controlled by the length of slot in the template 104 and so only one cutting tool 1 is still required.

## Claims

1. A surgical cutting tool, the cutting tool comprising:
a proximal end portion, the proximal end portion providing a connection location;
a distal end portion, the distal end portion providing a cutting head;
wherein the cutting head includes a body element, the body element includes a toothed section and the toothed section is provided with a plurality of teeth elements, at least two rows of teeth elements being provided, a plurality of the teeth elements each including an inclined face on a distal side or proximal side of the teeth element and a further inclined face on the other side of the teeth element, the inclined face of a teeth element facing across the cutting head and also face along the cutting head.

2. The surgical cutting tool of claim 1, wherein a connection portion of the cutting head is provided intermediate an intermediate portion of the cutting tool and the cutting head, the connection portion having an angular offset compared with the longitudinal axis of the cutting tool.

3. The surgical cutting tool of claim 1 or claim 2, wherein the body element is provided with a projecting element extending from at least one side of the cutting tool.

4. The surgical cutting tool of claim 3, wherein the projecting element extends from a side surface of the body element.

5. The surgical cutting tool of claim 3 or claim 4, wherein the projecting element is provided with a lower surface which includes a planar portion and wherein the planar portion is substantially parallel or parallel to the longitudinal axis of the cutting tool and/or to the axis of reciprocation of the cutting tool in use.

6. The surgical cutting tool of claim 5, wherein the planar portion is substantially perpendicular or perpendicular to the adjacent side surface of the body element.

7. The surgical cutting tool of claim 5 or claim 6, wherein the distance between the planar portion of the lower surface and one or more of the tips of the teeth elements, in use, controls the cutting depth of the cutting tool.

8. The surgical cutting tool of any preceding claim 1, wherein the inclined face of a teeth element and the further inclined face of that teeth element face across the cutting head and also face along the cutting head, in opposing directions and/or
wherein the plurality of teeth elements define a gap between the teeth elements in a cross-sectional profile across a plane, the plane being perpendicular to the longitudinal axis of the cutting tool, the gap extending from a distal side of the cutting head to a proximal side of the cutting head, when viewed along the longitudinal axis.

9. The surgical cutting tool of any preceding claim, wherein the body element is flared outwardly along the longitudinal axis of the cutting tool, between an upper portion of the body element and a lower portion of the body element and/or
wherein greater than 50% of the teeth elements are provided in a common plane, the common plane being substantially parallel or parallel to a plane in which the cutting tool reciprocates, in use.

10. The surgical cutting tool of any preceding claim, wherein one or more of the teeth elements of one row are:
a) at least partially interdigitated with one or more of the teeth elements of another row; or
b) at least partially overlap with one or more of the teeth elements of another row; or
c) staggered relative to one or more of the teeth elements of another row.

11. The surgical cutting tool of claim 8 or any claim depending thereupon, wherein the gap extends between the teeth elements of one row of teeth elements and the teeth elements of another row of teeth elements and/or
wherein the gap is a gullet and/or
wherein the gap is a single gap only.

12. The surgical cutting tool of claim 8 or any claim depending thereupon, wherein the gap is defined by the projection of a portion of the edge of the teeth elements in one row of teeth elements and the projection of a portion of the edge of the teeth elements in another row of teeth elements and the gap is further defined by the intersection of the projection of the portion of the edge of the teeth elements in one row of teeth elements and the projection of the intersection of the portion of the edge of the teeth elements in another row of teeth elements and/or
wherein, when viewed in the plane, the gap is an inverted V-shape.

13. The surgical cutting tool of claim 1 or any claim depending thereupon, wherein the body element is not flared perpendicular to the longitudinal axis of the cutting tool and/or
wherein a plurality of the teeth elements have a tip and the tips of the plurality of teeth elements are provided in the common plane.

14. A kit comprising:
a cutting tool, the cutting tool comprising:
a proximal end portion, the proximal end portion providing a connection location;
a distal end portion, the distal end portion providing a cutting head;
wherein the cutting head includes a body element, the body element includes a toothed section and the toothed section is provided with a plurality of teeth elements, at least two rows of teeth elements being provided, a plurality of the teeth elements each including an inclined face on a distal side or proximal side of the teeth element and a further inclined face on the other side of the teeth element, the inclined face of a teeth element facing across the cutting head and also face along the cutting head; and
one or more further surgical components.

15. The kit of claim 19, wherein the one or more surgical components includes a cutting template provided with a through slot and/or
wherein the cutting template comprises an at least partially planar upper surface or superior surface, the upper surface or superior surface being configured to contact a projecting element provided on the cutting tool.
